# EUROPÄISCHE PATENTSCHRIFT

(11) **EP 1 204 414 B1**
(45) Veröffentlichungstag und Bekanntmachung des Hinweises auf die Patenterteilung: **11.07.2007**
(21) Anmeldenummer: 00954588.0
(22) Anmeldetag: 04.08.2000
(51) Int. Cl.: A61K 31/485, A61P 25/00

(54) **VERWENDUNG VON MORPHINDERIVATEN ALS ARZNEIMITTEL ZUR BEHANDLUNG VON NEUROPATISHCHEN PROBLEME**
USE OF MORPHINAN DERIVATES AS MEDICINES
UTILISATION DE DERIVES DE MORPHINANE COMME MEDICAMENTS

(30) Priorität: 18.08.1999 DE 19939044
(43) Veröffentlichungstag der Anmeldung: 15.05.2002
(73) Patentinhaber: Grünenthal GmbH, 52078 Aachen (DE)
(72) Erfinder: WNENDT, Stephan, D-52062 Aachen (DE); STRASSBURGER, Wolfgang, D-52146 Würselen (DE); BUSCHMANN, Helmut, D-52066 Aachen (DE); REISSMÜLLER, Elke, D-33617 Bielefeld (DE); KRÜGER, Thomas, D-52379 Langerwehe-Schlich (DE)
(86) Internationale Anmeldenummer: PCT/EP2000/007585
(87) Internationale Veröffentlichungsnummer: WO 2001/012195

(56) Entgegenhaltungen:
- WO-A-98/22467
- WO-A-98/31684
- US-A- 5 852 030
- DATABASE CAPLUS [Online] an:2000:140726 , 1998 UKAI: "development of neuropeptides..." XP002161946 & UKAI: "development of neuropeptides..." IKAGAKU OYO KENKYU ZAIDAN KENKYU HOKOKU, Bd. 17, 1998, Seiten 127-130, japan

## Beschreibung

Die Erfindung betrifft die Verwendung von Morphinanderivaten, sowie deren Basen oder Salze von physiologisch verträglichen Säuren als Regulatoren für das Nociceptin/Orphanin FQ-Ligand-ORL1-Rezeptor-System zur Herstellung eines Arzneimittels.

Das Heptadekapeptid Nociceptin/Orphanin FQ ist ein endogener Ligand des ORL1 (Opioid-Receptor-Like)-Rezeptors (Meunier et al., Nature 377, 1995, S. 532-535), der zu der Familie der Opioid Rezeptoren gehört und in vielen Regionen des Gehirnes und des Rückenmarkes zu finden ist (Mollereau et al., FEBS Letters, 341, 1994, S. 33-38, Darland et al., Trends in Neurosciences, 21, 1998, S. 215-221). Das Peptid ist durch eine hohe Affinität, mit einem K_{d}-Wert von annähernd 56 pM (Ardati et al., Mol. Pharmacol. 51, S. 816-824) und durch eine hohe Selektivität für den ORL1-Rezeptor gekennzeichnet. Der ORL1-Rezeptor ist homolog zu den µ, κ und δ Opioid-Rezeptoren und die Aminosäuresequenz des Nociceptin/Orphanin FQ Peptides weist eine starke Ähnlichkeit mit denen der bekannten Opioidpeptide auf. Die durch das Nociceptin/Orphanin FQ induzierte Aktivierung des Rezeptors führt über die Kopplung mit G_{i/o}-Proteinen zu einer Inhibierung der Adenylatcyclase (Meunier et al., Nature 377, 1995, S. 532-535). Auch auf der zellulären Ebene sind funktionelle Ähnlichkeiten der µ, κ und δ Opioid-Rezeptoren mit dem ORL1-Rezeptor in Bezug auf die Aktivierung des Kalium-Kanals (Matthes et al., Mol. Pharmacol. 50, 1996, S. 447-450; Vaughan et al., Br. J. Pharmacol. 117, 1996, S. 1609-1611) und der Inhibierung der L-, N- und P/Q-Typ-Kalzium-Kanäle vorhanden (Conner et al., Br. J. Pharmacol. 118, 1996, S. 205-207; Knoflach et al., J. Neuroscience 16, 1996, S. 6657-6664).

Das Nociceptin/Orphanin FQ Peptid zeigt nach intercerebroventicularer Applikation eine pronociceptive und hyperalgesische Aktivität in verschiedenen Tiermodellen (Reinscheid et al., Science 270, 1995, S. 792-794; Hara et al,. Br. J. Pharmacol. 121, 1997, S. 401-408). Diese Befunde können als Hemmung der stressinduzierten Analgesie erklärt werden (Mogil et al., Neurosci. Letters 214, 1996, S131-134; sowie Neuroscience 75, 1996, S. 333-337). In diesem Zusammenhang konnte auch eine anxiolytische Aktivität des Nociceptin/Orphanin FQ-Peptides nachgewiesen werden (Jenck et al., Proc. Natl. Acad. Sci. USA 94, 1997, 14854-14858).

Auf der anderen Seite konnte in verschiedenen Tiermodellen auch ein antinociceptiver Effekt von Nociceptin/Orphanin FQ, insbesondere nach intrathekaler Applikation, gezeigt werden. Nociceptin/Orphanin FQ hemmt die Aktivität Kainat- oder Glutamat-stimulierter Hinterwurzelganglienneuronen (Shu et al., Neuropeptides, 32, 1998, 567-571) oder Glutamat-stimulierter Rückenmarksneuronen (Faber et al., Br. J. Pharmacol., 119, 1996, S. 189-190); es wirkt antinociceptiv im Tail-Flick-Test in der Maus (King et al., Neurosci. Lett., 223, 1997, 113-116), im Fiexor-Reflex-Modell in der Ratte (Xu et al., NeuroReport, 7, 1996, 2092-2094) und im Formalin-Test an der Ratte (Yamamoto et al., Neuroscience, 81, 1997, S. 249-254). In Modellen für neuropathische Schmerzen konnte ebenfalls eine antinociceptive Wirkung von Nociceptin/Orphanin FQ nachgewiesen werden (Yamamoto and Nozaki-Taguchi, Anesthesiology, 87, 1997), die insofern besonders interessant ist, als das die Wirksamkeit von Nociceptin/Orphanin FQ nach Axotomie von Spinalnerven zunimmt. Dies steht im Gegensatz zu den klassischen Opioiden, deren Wirksamkeit unter diesen Bedingungen abnimmt (Abdulla and Smith, J. Neurosci., 18, 1998, S. 9685-9694).

Das Nociceptin/Orphanin FO-Ligand ORL1-Rezeptor-System ist außerdem noch an der Regulation weiterer physiologischer und pathophysiologischer Prozesse beteiligt. Hierzu gehören unter anderem Lernen und Gedächtnisbildung (Sandin et al., Eur. J. Neurosci., 9, 1997, S. 194-197; Manabe et al., Nature, 394, 1997, S. 577-581), Hörvermögen (Nishi et al., EMBO J., 16, 1997, S. 1858-1864), Nahrungsaufnahme (Pomonis et al., NeuroReport, 8, 1996, S. 369-371), Regulation des Blutdruckes (Gumusel et al., Life Sci., 60, 1997, S. 141-145; Campion und Kadowitz, Biochem. Biophys. Res. Comm., 234, 1997, S. 309-312), Epilepsie (Gutiérrez et al, Abstract 536.18, Society for Neuroscience, Vol 24, 28th Ann. Meeting, Los Angeles, November 7.-12, 1998) und Diurese (Kapista et al., Life Sciences,-60, 1997, PL 15-21).

Morphinanderivate, sowie Verfahren zu deren Herstellung sind aus den WO 98/22467, WO 95/31463 und WO 95/31464 bekannt. Diese Verbindungen werden als δ-selektive Opioid-Agonisten und Opioid-Antagonisten zur Behandlung bei Erkrankungen, wie z.B. Schock, Konstipation, mentalen Störungen, Eßstörungen, Verletzungen des zentralen Nervensystems, Alkoholismus und Immunfunktionsstörungen beschrieben.

US 5852030 offenbart Morphinan-Derivate mit einem ankondensierten Indol-Derivat, die potente δ-Opioid-Antagonisten sind und immunsuppressive, antiallergische, antiinflammatorische und Zell-schützende Eigenschaften aufweisen. WO 9822467 offenbart eine Synthese zur Herstellung solcher Indolomorphinane.

Aufgabe der vorliegenden Erfindung war es, Arzneimittel zur Verfügung zu stellen, die auf das Nociceptin/Orphanin-FQ-Ligand-ORL1-Rezeptor-System wirken und damit zur Behandlung von neuropathischen Schmerzen und/oder Anxiolyse und/oder Depression und/oder Harninkontinenz und/oder Tinitus und/oder Schwerhörigkeit und/oder Epilepsie und/oder Fettsucht und/oder Kachexie geeignet sind.

Überraschenderweise wurde nun gefunden, daß Morphinanderivate der nachstehenden allgemeinen Formel I einen Einfluß auf die Steuerung unterschiedlicher physiologischer und pathophysiologischer Prozesse zeigen, bei denen das Nociceptin/Orphanin-FQ Ligand-ORL1-Rezeptor-System involviert ist. Zu den genannten Prozessen zählen u.a. die Empfindung neuropathischer Schmerzen, Angstverhalten, Hören, Nahrungsaufnahme und Epilepsie.

Gegenstand der vorliegenden Erfindung ist daher die Verwendung von Morphinanderivaten der allgemeinen Formel I, 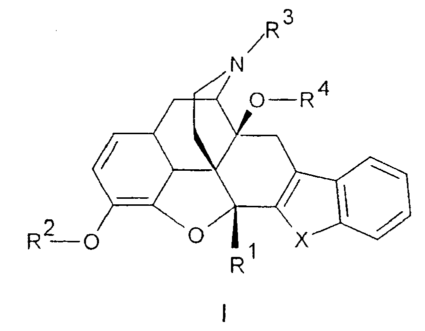 worin
R¹ = H, einen C₁₋₄-Alkyl- oder einen C₂₋₄-Alkenyl-Rest,
R² = H, einen C₁₋₁₈-Alkyl-, bevorzugt einen C₁₋₁₀-Alkyl-, einen C₂₋₁₈-Alkenyl-, bevorzugt einen C₂₋₁₀-Alkenyl-, einen Heterocyclyl- oder einen Aryl-Rest,
R³ = einen C₁₋₁₈-Alkyl-, bevorzugt einen C₁₋₁₀-Alkyl-, einen C₂₋₁₈-Alkenyl-, bevorzugt einen C₂₋₁₀-Alkenyl-, einen über eine C₁₋₄-Alkylen-Gruppe gebundenen C₃₋₇-Cycloalkyl-, Aryl- oder Heterocyclyl-Rest oder einen über eine C₂₋₄-Alkenylen-Gruppe gebundenen C₃₋₇-Cycloalkyl-, Aryl- oder Heterocyclyl-Rest
R⁴ = H, einen C₁₋₁₈-Alkyl-, bevorzugt einen C₁₋₁₀-Alkyl-, einen C₂₋₁₈-Alkenyl-, bevorzugt einen C₂₋₁₀-Alkenyl-, einen über eine C₁₋₄-Alkylen-Gruppe gebundenen C₃₋₇-Cycloalkyl- oder Aryl- oder einen über eine C₂₋₄-Alkenylen-Gruppe gebundenen C₃₋₇-Cycloalkyl- oder Aryl-Rest..
und
X = O oder NR⁵ mit
R⁵ = H, einen C₁₋₁₈-Alkyl-, bevorzugt einen C₁₋₁₀-Alkyl-, einen C₂₋₁₈-Alkenyl-, bevorzugt einen C₂₋₁₀-Alkenyl-, einen über eine C₁₋₄-Alkylen-Gruppe gebundenen C₃₋₇-Cycloalkyl-, Aryl- oder Heterocyclyl-Rest oder einen über eine C₂₋₄-Alkenylen-Gruppe gebundenen C₃₋₇-Cycloalkyl-, Aryl- oder Heterocyclyl-Rest
bedeuten,
und/oder deren Enantiomeren, Diastereomeren, Basen oder Salze von physiologisch verträglichen Säuren zur Herstellung eines Arzneimittels zur Behandlung von: neuropathischen Schmerzen und/oder Anxiolyse und/oder Depression und/oder Harninkontinenz und/oder Tinitus und/oder Schwerhörigkeit und/oder Epilepsie und/oder Fettsucht und/oder Kachexie.

Unter Alkyl-Resten werden auch mindestens einfach, vorzugsweise mit Halogen, besonders bevorzugt mit Fluor, substituierte Kohlenwasserstoffe verstanden. Enthalten diese mehr als einen Substituenten, so können diese gleich oder verschieden sein.
Vorzugsweise sind die Alkyl-Reste Methyl, Ethyl, Propyl, 1-Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Heptyl, Nonyl oder Decanyl.

Unter Alkenyl-Resten werden auch mindestens einfach, vorzugsweise mit Halogen, besonders bevorzugt mit Fluor, substituierte Kohlenwasserstoffe verstanden, die mindestens eine Doppelbindung aufweisen. Enthält der Alkenyl-Rest mehr als einen Substituenten, so können diese gleich oder verschieden sein. Vorzugsweise sind die Alkenyl-Reste 2-Propenyl, 2-Butenyl, 1-Methyl-2-propenyl oder 2-Methyl-2-propenyl.

Unter einem Aryl-Rest werden auch mindestens einfach mit einem OH-, einem Halogen-, vorzugsweise F- und/oder Cl-, einem CF₃-, einem C₁₋₆-Alkyl-, einem C₁₋₆-Alkoxy-, einem C₁₋₇-Cycloalkoxy-, einem C₃₋₇-Cycloalkyl-, einem C₂₋₆-Alkylen- oder Phenyl-Rest substituierte Phenyle oder Naphtyl-Reste verstanden. Die Phenylreste können auch mit weiteren Ringen kondensiert sein.

Unter einem Heterocyclyl-Rest werden sowohl gesättigte, wie auch ungesättigte heterocyclische Verbindungen, bevorzugt 5-7-gliedrige heterocyclische Verbindungen verstanden, die mindestens ein Heteroatom, vorzugsweise Stickstoff, Sauerstoff und/oder Schwefel, besonders bevorzugt Stickstoff und/oder Sauerstoff enthalten. Vorzugsweise sind die gesättigten Heterocyclen 1,4-Dioxan, Tetrahydrofuran oder 1,4 Thioxan. Vorzugsweise sind die ungesättigten Heterocyclen Furan, Thiophen, Pyridin, Pyrimidin, Thiazol, Oxazol, Isoxazol, Pyridazin, Pyrazin, Chinolin, Isochinolin. Phthalazin oder Chinazolin.

Bevorzugt sind Morphinanderivate der allgemeinen Formel I, in denen R¹ = H oder ein C₁₋₄-Alkyl-Rest, R² = H oder ein C₁₋₄-Alkyl-Rest, R³ = ein C₁₋₄-Alkyl- oder ein C₂₋₄-Alkenyl-Rest, R⁴ = H, ein C₁₋₄-Alkyl-, ein über eine C₁₋₄-Alkylen-Gruppe gebundener C₃₋₇-Cycloalkyl- oder Aryl-Rest ist und X = NR⁵, worin R⁵ = ein C₁₋₁₀-Alkyl-, ein C₂₋₁₀-Alkenyl, ein über eine C₁₋₄-Alkylen-Gruppe gebundener C₃₋₇-Cycloalkyl- oder Aryl- oder ein über eine C₂₋₄-Alkenylen-Gruppe gebundener C₃₋₇-Cycloalkyl- oder Aryl-Rest ist.

Ebenfalls bevorzugt sind Morphinanderivate der allgemeinen Formel I, in denen R¹ = H oder ein C₁₋₄-Alkyl-Rest, R² = H, R³ = ein C₁₋₄-Alkyl- oder ein C₂₋₄-Alkenyl-Rest, R⁴ = H, ein C₁₋₄-Alkyl-, oder ein über eine C₁₋₄-Alkylen-Gruppe gebundener C₃₋₇-Cycloalkyl- oder Aryl-Rest ist und X = NR⁵, worin R⁵ = ein C₂₋₁₀-Alkenyl, ein über eine C₁₋₄-Alkylen-Gruppe gebundener C₃₋₇-Cycloalkyl- oder Aryl- oder ein über eine C₂₋₄-Alkenylen-Gruppe gebundener Aryl-Rest ist.

Besonders bevorzugt sind Morphinanderivate der allgemeinen Formel I, in denen R¹ = H, R² = H, R³ = ein C₂₋₄-Alkenyl-Rest, R⁴ = H und X = NR⁵, worin R⁵ = ein über eine C₁₋₄-Alkylen-Gruppe gebundener Aryl- oder ein über eine C₂₋₄-Alkenylen-Gruppe gebundener Aryl-Rest ist.

Ganz besonders bevorzugt sind die nachstehenden Morphinanderivate
17-Allyl-6,7-didehydro-4,5α-epoxy-3,14-dihydroxy-1'-(o-chlorbenzyl) indolo [6,7:2,'3']-morphinan-Hydrochlorid,
17-Allyl-6,7-didehydro-4,5α-epoxy-3-hydroxy-14-(m-chlormethoxyphenyl)1'-(m-chlorbenzyl) indolo [6,7:2,'3']-morphinan-Hydrochlorid,
oder
17-Cyclopropylmethyl-6,7-didehydro-4,5α-epoxy-3-hydroxy-14-(o-chlormethoxyphenyl)1'-(o-chlorbenzyl) indolo [6,7:2,'3']-morphinan-Hydrochlorid
beziehungsweise
17-Cyclopropylmethyl-6,7-didehydro-4,5α-epoxy-3-hydroxy-14-(methoxynaphthalin)1'-(β-methyl-naphthalin) indolo [6,7:2,'3']-morphinan-Hydrochlorid,
oder
17-Cyclopropylmethyl-6,7-didehydro-4,5α-epoxy-14-hydroxy-3-benzyloxy-1'-(p-methoxycarbonylmethylphenyl)-indolo [6,7:2,'3']-morphian-Hydrochlorid

Zur Zubereitung entsprechender pharmazeutischer Formulierungen werden neben mindestens einem Morphinanderivat der Formel I Trägermaterialien, Füllstoffe, Lösungsmittel, Verdünnungsmittel, Farbstoffe und/oder Bindemittel eingesetzt. Die Auswahl der Hilfsstoffe sowie die einzusetzenden Mengen derselben hängt davon ab, ob das Arzneimittel oral, intravenös, intraperitoneal, intradermal, intramuskulär, intranasal, buccal oder örtlich appliziert werden soll. Für orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen der Formel I in einem Depot, in gelöster Form oder in einem Pflaster, gegebenenfalls unter Zusatz von die Hautpenetration fördernden Mittel, sind geeignete perkutane Applikationszubereitungen. Oral oder perkutan anwendbare Zubereitungsformen können die erfindungsgemäßen Verbindungen der Formel I verzögert freisetzen.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,1 bis 1 mg/kg wenigstens eines Morphinanderivates der Formel I appliziert.

### Molekularpharmakologische Untersuchungen

Die erfindungsgemäßen Morphinanderivate der allgemeinen Formel I wurden mit einem Reportergensystem als ORL1-Rezeptor-Agonisten bzw. als ORL1-Rezeptor-Antagonisten identifiziert. Dieses Reportergensystem basiert auf der Expression des humanen ORL1-Rezeptors in CHO-K1 Zellen, die ein cAMPsensitives Luziferasegen tragen (Wnendt et al., Regulatory Peptides, 80, 1999, S. 127). Durch Forskolin, einem Aktivator der Adenylatzyclase, wird die Bildung von cAMP induziert. Da der ORL1-Rezeptor bei Bindung des natürlichen Liganden Nociceptin/Orphanin FQ die Bildung von cAMP durch Hemmung der Adenylatcyclase reduziert, ist die Wirkung einer Substanz als Agonist oder Antagonist an dem ORL-Rezeptor durch eine Veränderung der cAMP-abhängigen Luziferasebildung nachweisbar.

ORL1-Rezeptor-Agonisten zeigen die gleiche Wirkung wie der natürliche Ligand Nociceptin/Orphanin FQ. Auch sie führen durch die Bindung an den ORL1-Rezeptor konzentrationsabhängig zu einer Hemmung der Luziferasebildung. Die Potenz, als IC₅₀ Wert angegeben, zeigt an, bei welcher Konzentration die halbmaximale Wirkung des Agonisten erreicht wird.

Die ORL1-Rezeptor-Antagonisten konkurrieren mit dem natürlichen Liganden Nociceptin/Orphanin FQ um die Bindung an den ORL1-Rezeptor. In dem Maße wie der Antagonist an den ORL-1 Rezeptor bindet, wird die durch den Agonisten verursachte Hemmung der Luziferase-Bildung aufgehoben. Die Dosis-Wirkungskurve des natürlichen Liganden Nociceptin/Orphanin FQ wird in höhere Konzentrationsbereiche verschoben. Die Wirkung der Antagonisten wird durch den pK_{B} Wert beschrieben. Diese logarithmische Größe gibt die Konzentration an, bei der die Potenz des natürlichen Liganden Nociceptin/Orphanin FQ um den Faktor 2 verringert wird (Kenakin, T, Pharmacological Analysis of Drug-Receptor Interaction, 3. Auflage, Lippincott-Raven, 1997, Philadelphia, New York).

Das Reportergensystem wurde wie folgt aufgebaut:

Eine den ORL1-Rezeptor kodierende cDNA wurde unter Verwendung von Standardmethoden aus THP-1 Zellen (European Cell Culture Collection, Porton Down, Grossbritannien), einer humanen, monozytären Zellinie, kloniert und in das Plasmid pZeoSV (Invitrogen, Leek, Niederlande) integriert. Dieses Plasmid, pZeoORL17, wurde zur Transfektion einer Zellinie eingesetzt, CHO-K1/pSE66/K9, die das cAMP-Reporterplasmid pSE66 enthält.
Das Plasmid pSE66 wurde auf der Basis des Plasmids pMAMneo-LUC (Clontech, Palo Alto, Kalifornien, USA) konstruiert, indem der RSV Promoter dieses Plasmides durch eine Promoterregion ersetzt wurde, bei der sechs CRE-Elemente (cAMP-responsive Elemente, Comb et al., Nature, 323, 1986, S. 353-356; Montminy et al., Proc. Natl. Acad. Sci. USA, 83, 1986, S. 6682-6686; Short et al., J. Biol. Chem., 261, 1986, S. 9721-9726) stromauf zu einem Promoter liegen. Der SV40 Promoter stammt aus dem Plasmid pGL2-Promoter (Promega, Madison, Wisconsin, USA). Die auf diese Weise konstruierte Promoterregion kontrolliert im Plasmid pSE66 die Expression des Luziferase-Gens aus pMAMneo-LUC. Darüberhinaus trägt pSE66 ein G418-Resistenz-Gen unter Kontrolle eines weiteren SV40 Promoters. Die Zellinie CHO-K1 (European Cell Culture Collection, Porton Down, Grossbritannien) wurde stabil mit pSE66 transfiziert und der Klon CHO-K1/pSE66/K9 für die Transfektion mit pZeoORL17 ausgewählt. Monoklonale pZeoORL17-Transformanten wurden in Nutrient Mixture F-12 (Ham) mit Glutamin (Gibco-BRL, Weiterstadt, Deutschland), ergänzt mit 50 µg/ml G418 (Gibco-BRL, Weiterstadt, Deutschland) und 200 µg/ml Zeocin (Invitrogen, Leek, Niederlande), selektiert.
Nociceptin/Orphanin FQ sensitive Klone wurden identifiziert, indem je 20.000 Zellen eines Klons in einer 96-Well-Mikrotiterplatte in einem Volumen von 100 µl für 6 Stunden mit 1 µM Forskolin (RBI, Deisenhofen) in An- bzw. Abwesenheit von 10 µM Nociceptin/Orphanin FQ inkubiert und anschließend mit dem Luciferase-Detection-Kit (Roche, Mannheim) vermessen wurden (Ford und Leach, Methods in Molecular Biology, 102, 1998, S. 3-20).
Der Klon CHO-K1/pSE66/K9/pZeoORL17/K21 wurde zur Analyse von Testverbindungen ausgewählt, wobei die Testverbindungen in Dimethylsulfoxid gelöst wurden und die Endkonzentration an Dimethylsulfoxid auf 1 % (v/v) festgelegt wurde. Der Test von Antagonisten wurde in Anwesenheit von 10 nM Nociceptin/Orphanin FQ durchgeführt.

Desweiteren wurde mit den erfindungsgemäßen Verbindungen ein Rezeptorbindungsassay mit ³H - Nociceptin/Orphanin FQ mit Membranen von rekombinanten CHO-ORL1 Zellen durchgeführt. Dieses Testsystem wurde gemäß der von Ardati et al. (Mol. Pharmacol., 51, 1997, S. 816-824) vorgestellten Methode durchgeführt. Die Konzentration von ³H-N/OFQ betrug bei diesen Versuchen 0.5 nM. Die Bindungsassays wurden mit je 20 µg Membranprotein je 200 µl Ansatz in 50 mM Hepes, pH 7,4, 10 mM MgCl₂ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei Raumtemperatur und anschliessende Messung im Szintillationscounter Trilux (Wallac, Finnland), bestimmt. Die Affinität wird als Kᵢ-Wert angegeben.

Die Herstellung der Morphinanderivate der allgemeinen Formel I ist in WO 95/31463 beziehungsweise WO 95/31464 beschrieben.

### Beispiele:

Die folgenden Beispiele dienen der Erläuterung der Erfindung, schränken aber den allgemeinen Erfingungsgedanken nicht ein.

Von jeder dieser beispielhaften Verbindungen 1 bis 7 wurde gemäß den angegebenen molekularpharmakologischen Untersuchungen die agonistische bzw. die antagonistische Wirkung auf den ORL1-Rezeptor bestimmt. Die entsprechenden IC₅₀- bzw pK_{B}-Werte sind in der Tabelle 1 angegeben.

**Tabelle 1:**

| Beispiel | ORL1-Antagonisten | PK_{B} ORL1 Reportergenassay | Kᵢ (nM) ORL1 Bindungsassay |
|---|---|---|---|
| 1 | 17-Allyl-6,7-didehydro-4,5α-epoxy-3,14-dihydroxy-1'-(o-chlorbenzyl)indolo [6,7:2,'3']-morphinan-Hydrochlorid | 7,37 | 449 |
| 2 | 17-Allyl-6,7-didehydro-4,5α-epoxy-3-hydroxy-14-(m-chlormethoxyphenyl)1'-(m-chlorbenzyl) indolo [6,7:2,'3']-morphinan-Hydrochlorid | 6,99 | 1079 |
| 3 | 17-Cyclopropylmethyl-6,7-didehydro-4,5α-epoxy-3-hydroxy-14-(o-chlormethoxyphenyl)1'-(o-chlorbenzyl) indolo [6,7:2,'3']-morphinan-Hydrochlorid | 6,39 | 718 |
| 4 | 17-Cyclopropylmethyl-6,7-didehydro-4,5α-epoxy-3-hydroxy-14-(methoxynaphthalin)1'-(β-methylnaphthalin) indolo [6,7:2,'3')-morphinan-Hydrochlorid | 31,2 | 982 |
| 5 | 17-Cyclopropylmethyl-6,7-didehydro-4,5α-epoxy-14-hydroxy-3-benzyloxy-1'-(p-methoxycarbonylmethylphenyl)-indolo [6,7:2,'3']-morphian-Hydrochlorid | 25,8 | Nicht bestimmt |

Die Morphinanderivate der allgemeinen Formel sind durch folgende Methoden synthetisiert worden:

Durch Behandlung von Thebain der Formel II 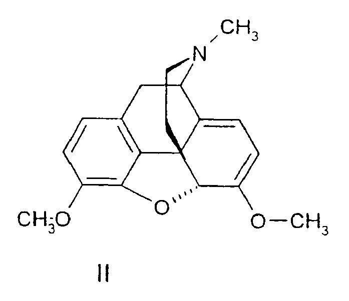 mit Dialkylsulfaten, Fluorsulfonsäurealkylestern, Alkylsulfonsäurealkylester, Arylsulfonsäurealkylester, Alkylhalogeniden, Arylalkylhalogeniden, Alkylsulfonsäurearylester, Arylsulfonsäurearylester, Arylalkenyl-halogeniden oder Chloroformiaten in Lösungsmitteln wie Tetrahydrofuran, oder Diethylether unter Verwendung einer starken Base wie z. B. n-Butyllithium, Lithiumdiethylamid oder Lithiumdiisopropylamid bei tiefen Temperaturen (-20 bis -80 °C) konnten Verbindungen der Formel III erhalten werden (Boden et al., J. Org. Chem. 1982, 47, 1347-1349; H. Schmidhammer et al., Helv. Chim. Acta 1988, 71, 642-647), 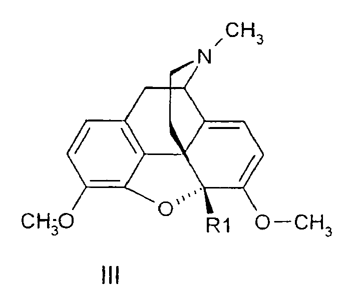 worin R¹ = H, einen C₁₋₄-Alkyl- oder einen C₂₋₄-Alkenylrest bedeutet.

Durch Reaktion der substituierten Thebain-Derivate der Formel III mit Perameisensäure oder m-Chlor-perbenzoesäure bei Temperaturen zwischen 0 und 60 °C konnten die Hydroxycodeinon-Derivate der Formel IV erhalten werden (H. Schmidhammer et al., Helv. Chim. Acta 1988, 71, 1801-1804).

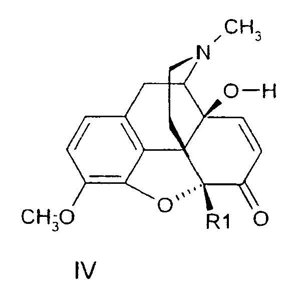

Durch Behandlung der Hydroxycodeinon-Derivate der Formel IV mit Dialkylsulfaten, Alkylhalogeniden, Cycloalkylhalogeniden, Cycloalykylalkenylhalogenide, Alkenylhalogeniden, Arylalkylhalogeniden, Arylalkenyl-halogeniden oder Chloroformiaten in Lösungsmitteln wie N,N-Dimethylformamid oder Tetrahydrofuran unter Verwendung starker Basen wie Natriumhydrid oder Kaliumhydrid konnten Verbindungen der Formel V hergestellt werden, 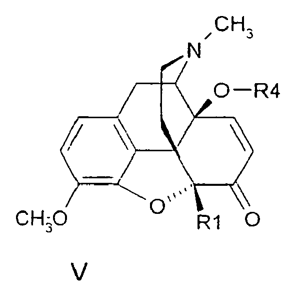 worin R¹ die Bedeutung wie in Formel III hat und R⁴ einen C₁₋₁₈-Alkyl, bevorzugt einen C₁₋₁₀-Alkyl, einen C₂₋₁₈-Alkenyl-, bevorzugt einen C₂₋₁₀-Alkenyl, einen über eine C₁₋₄-Alkylengruppe gebundenen C₃₋₇-Cycloalkyl- oder Aryl- oder einen über eine C₂₋₄-Alkenylen-Gruppe gebundenen C₃₋₇-Cycloalkyl- oder Aryl-Rest bedeutet.

Durch katalytische Hydrierung unter Verwendung von Katalysatoren wie Palladium auf Aktivkohle in Lösungsmitteln wie Methanol, Ethanol oder Eisessig konnten die Verbindungen der Formel V in Verbindungen der Formel VI überführt werden, 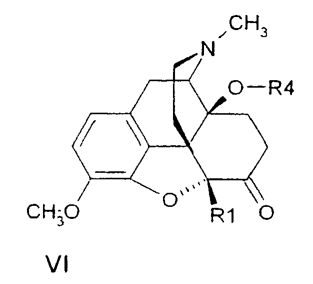 worin R¹ = H, einen C₁₋₄-Alkyl- oder einen C₂₋₄-Alkenylrest und R⁴ einen C₁₋₁₈-Alkyl, bevorzugt einen C₁₋₁₀-Alkyl, einen C₂₋₁₈-Alkenyl-, bevorzugt einen C₂₋₁₀-Alkenyl, einen über eine C₁₋₄-Alkylengruppe gebundenen C₃₋₇-Cycloalkyl- oder Aryl- oder einen über eine C₂₋₄-Alkenylen-Gruppe gebundenen C₃₋₇-Cycloalkyl- oder Aryl-Rest bedeuten.
In an sich bekannter Weise konnten aus Verbindungen der Formel VI durch N-Demethylierung, vorzugsweise unter Verwendung von Chloroformiaten und anschließender reduktiver Addition, Verbindungen der Formel VII erhalten werden, 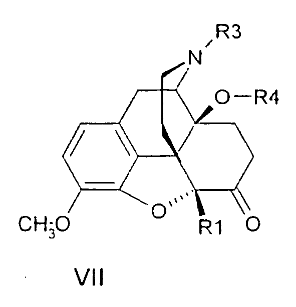 worin R¹ und R⁴ die Bedeutung wie in Formel VI haben und R³ einen C₁₋₁₈-Alkyl-, bevorzugt einen C₁₋₁₀-Alkyl-, einen C₂₋₁₈-Alkenyl, bevorzugt einen C₂₋₁₀-Alkenyl, einen über eine C₁₋₄-Alkylen-Gruppe gebundenen C₃₋₇-Cyclo-alkyl-, Aryl- oder Heterocyclyl-Rest oder einen über eine C₂₋₄-Alkenylen-Gruppe gebundenen C₃₋₇-Cycloalkyl-, Aryl oder Heterocyclyl-Rest bedeutet.

Durch eine Etherspaltung unter Verwendung von Bortribromid in Lösungsmitteln wie Dichlormethan oder Chloroform bei Temperaturen zwischen -10 und +10 °C, oder durch Erhitzen unter Rückfluß in 48 %iger Bromwasserstoffsäure oder unter Verwendung anderer wohlbekannter Etherspaltungsreagenzien konnten Substanzen der Formel VIII erhalten werden, 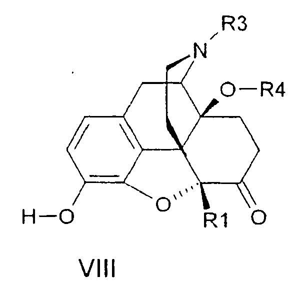 worin R¹, R³ und R⁴ die Bedeutung wie in Formel VII haben.

Durch Reaktion der phenolischen Hydroxylfunktion mit Basen wie Natriumhydrid in Lösungsmitteln wie Tetrahydrofuran oder N,N-Dimethylformamid und anschließender Zugabe von Alkylhalogeniden, Alkenylhalogeniden, Heterocyclylhalogeniden oder Arylhalogeniden ließen sich Verbindungen der Formel IX erhalten (H. Schmidhammer et al., J. Med. Chem. 1995, 38, 3071-3077), 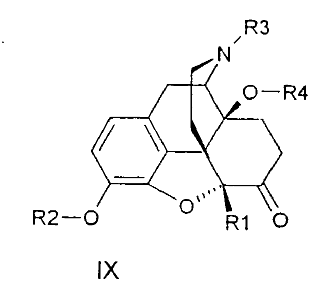 worin R¹, R³ und R⁴ die Bedeutung wie in Formel VII haben und R² einen C₁₋₁₈-Alkyl-, bevorzugt einen C₁₋₁₀-Alkyl-, einen C₂₋₁₈-Alkenyl, bevorzugt einen C₂₋₁₀-Alkenyl-, einen Heterocyclyl- oder Aryl-Rest bedeutet.

Verbindungen der Formel IX konnten durch Umsetzung mit Phenylhydrazin-Hydrochlorid in Gegenwart einer Säure, bevorzugt unter Verwendung von Methansulfonsäure, Schwefelsäure oder Salzsäure, im Sinne der Indolsynthese nach Fischer in Verbindungen der allgemeinen Formel I überführt werden, worin X = NH bedeutet und die übrigen Reste R¹ bis R⁴ die Bedeutung wie in Formel IX haben (P.S. Porthogese et al., J. Med.. Chem. 1988, 31, 281-282, H. Schmidhammer et al, J. Med. Chem. 1990, 33, 1200-1206).

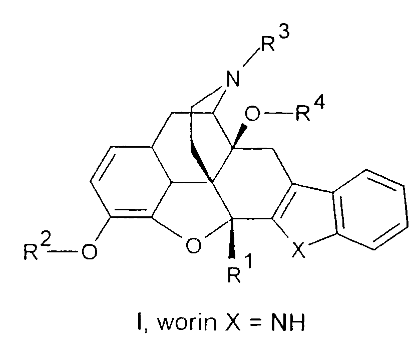

Verbindungen der allgemeinen Formel I, worin X = NR⁵ bedeutet, konnten entweder durch Kondensation von Verbindungen der Formel IX mit Bocgeschützten N-substituierten N-Phenylhydrazinen der Formel X, vorzugsweise durch Erhitzen unter Rückfluß in methanolischer Salzsäure erhalten werden (P.S. Porthogese et al. J. Med. Chem. 1994, 37, 1882-1885), oder sind durch Additionsreaktionen, ausgehend von Verbindungen der Formel I, worin X = NH bedeutet, in an sich bekannter Weise erhalten worden.

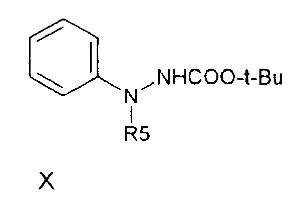

Verbindungen der Formel IX konnten durch Umsetzung mit O-Phenylhydroxylamin-Hydrochlorid in Gegenwart einer Säure, bevorzugt unter Verwendung von Methansulfonsäure, Schwefelsäure oder Salzsäure, in Verbindungen der allgemeinen Formel I überführt werden, worin X = O bedeutet und die übrigen Reste R¹ bis R⁴ die Bedeutung wie in Formel IX haben (P.S. Porthogese et al., J. Med.. Chem. 1988, 31, 281-282).

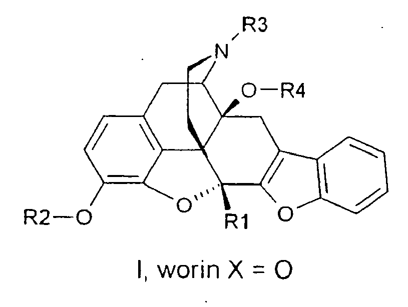

## Patentansprüche

1. Verwendung von Morphinanderivaten der allgemeinen Formel 1, 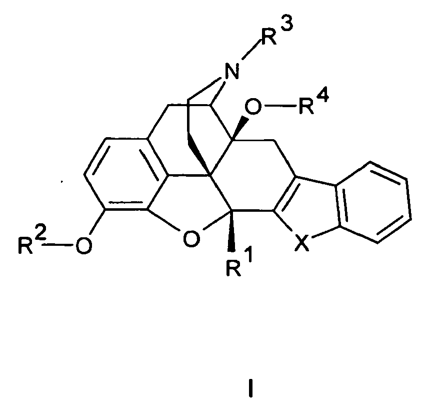 worin
R¹ = H, einen C₁₋₄-Alkyl- oder einen C₂₋₄-Alkenyl-Rest,
R² = H, einen C₁₋₁₈-Alkyl-, bevorzugt einen C₁₋₁₀-Alkyl-, einen C₂₋₁₈-Alkenyl-, bevorzugt einen C₂₋₁₀-Alkenyl-, einen Heterocyclyl- oder einen Aryl-Rest,
R³ = einen C₁₋₁₈-Alkyl-, bevorzugt einen C₁₋₁₀-Alkyl-, einen C₂₋₁₈-Alkenyl-, bevorzugt einen C₂₋₁₀-Alkenyl-, einen über eine C₁₋₄-Alkylen-Gruppe gebundenen C₃₋₇-Cycloalkyl-, Aryl- oder Heterocyclyl-Rest oder einen über eine C₂₋₄-Alkenylen-Gruppe gebundenen C₃₋₇-Cycloalkyl-, Aryl- oder Heterocyclyl-Rest
R⁴ = H, einen C₁₋₁₈-Alkyl-, bevorzugt einen C₁₋₁₀-Alkyl-, einen C₂₋₁₈-Alkenyl-, bevorzugt einen C₂₋₁₀-Alkenyl-, einen über eine C₁₋₄-Alkylen-Gruppe gebundenen C₃₋₇-Cycloalkyl- oder Aryl- oder einen über eine C₂₋₄-Alkenylen-Gruppe gebundenen C₃₋₇-Cycloalkyl- oder Aryl-Rest,
und
X = O oder NR⁵ mit
R⁵ = H, einen C₁₋₁₈-Alkyl-, bevorzugt einen C₁₋₁₀-Alkyl-, einen C₂₋₁₈-Alkenyl-, bevorzugt einen C₂₋₁₀-Alkenyl-, einen über eine C₁₋₄-Alkylen-Gruppe gebundenen C₃₋₇-Cycloalkyl-, Aryl- oder Heterocyclyl-Rest oder einen über eine C₂₋₄-Alkenylen-Gruppe gebundenen C₃₋₇-Cycloalkyl-, Aryl- oder Heterocyclyl-Rest
bedeuten,
und/oder deren Enantiomeren, Diastereomeren, Basen oder Salze von physiologisch verträglichen Säuren zur Herstellung eines Arzneimittels zur Behandlung von:
neuropathischen Schmerzen und/oder Anxiolyse und/oder Depression und/oder Harninkontinenz und/oder Tinitus und/oder Schwerhörigkeit und/oder Epilepsie und/oder Fettsucht und/oder Kachexie.

2. Verwendung nach Anspruch 1, **dadurch gekennzeichnet, daß** als Morphinanderivate
17-Allyl-6,7-didehydro-4,5α-epoxy-3,14-dihydroxy-1'-(o-chlorbenzyl)indolo [6,7:2,'3']-morphinan-Hydrochlorid,
17-Allyl-6,7-didehydro-4,5α-epoxy-3-hydroxy-14-(m-chlormethoxyphenyl)1'-(m-chlorbenzyl) indolo [6,7:2,'3']-morphinan-Hydrochlorid,
17-Cyclopropyl-6,7-didehydro-4,5α-epoxy-3-hydroxy-14-(o-chlormethoxyphenyl)1'-(o-chlorbenzyl) indolo [6,7:2,'3']-morphinan-Hydrochlorid,
17-Cyclopropyl-6,7-didehydro-4,5α-epoxy-3-hydroxy-14-(methoxynaphthalin)1'-(β-methyl-naphthalin) indolo [6,7:2,'3']-morphinan-Hydrochlorid,
und/oder
17-Cyclopropylmethyl-6,7-didehydro-4,5α-epoxy-14-hydroxy-3-benzyloxy-1'-(p-methoxycarbonylmethylphenyl)-indolo [6,7:2,'3']-morphian-Hydrochlorid
eingesetzt werden.

## Claims

1. Use of morphinan derivatives of the general formula I 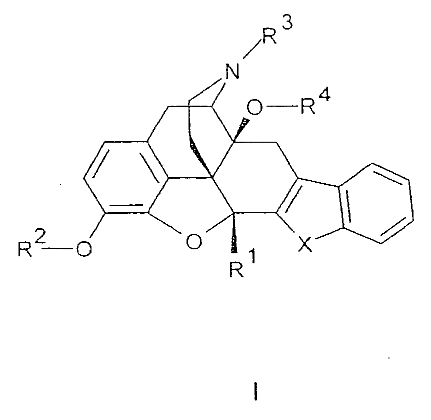 wherein
R¹ denotes H, a C₁₋₄ alkyl radical or a C₂₋₄ alkenyl radical,
R² denotes H, a C₁₋₁₈ alkyl radical, preferably a C₁₋₁₀ alkyl radical, a C₂₋₁₈ alkenyl radical, preferably a C₂₋₁₀ alkenyl radical, a heterocyclyl radical or an aryl radical,
R³ denotes a C₁₋₁₈ alkyl radical, preferably a C₁₋₁₀ alkyl radical, a C₂₋₁₈ alkenyl radical, preferably a C₂₋₁₀ alkenyl radical, a C₃₋₇ cycloalkyl, aryl or heterocyclyl radical bound via a C₁₋₄ alkylene group, or a C₃₋₇ cycloalkyl, aryl or heterocyclyl radical bound via a C₂₋₄ alkenylene group,
R⁴ denotes H, a C₁₋₁₈ alkyl radical, preferably a C₁₋₁₀ alkyl radical, a C₂₋₁₈ alkenyl radical, preferably a C₂₋₁₀ alkenyl radical, a C₃₋₇ cycloalkyl or aryl radical bound via a C₁₋₄ alkylene group, or a C₃₋₇ cycloalkyl or aryl radical bound via a C₂₋₄ alkenylene group,
and
X denotes O or NR⁵ where
R⁵ denotes H, a C₁₋₁₈ alkyl radical, preferably a C₁₋₁₀ alkyl radical, a C₂₋₁₈ alkenyl radical, preferably a C₂₋₁₀ alkenyl radical, a C₃₋₇ cycloalkyl, aryl or heterocyclyl radical bound via a C₁₋₄ alkylene group, or a C₃₋₇ cycloalkyl, aryl or heterocyclyl radical bound via a C₂₋₄ alkenylene group,
and/or their enantiomers, diastereomers, bases or salts of physiologically compatible acids, for the production of a medicament for treating neuropathic pain and/or anxiolysis and/or depression and/or urinary incontinence and/or tinnitus and/or impaired hearing and/or epilepsy and/or obesity and/or cachexia.

2. Use according to claim 1, **characterised in that** the following are used as morphinan derivatives
17-allyl-6,7-didehydro-4,5α-epoxy-3,14-dihydroxy-1'-(O-chlorobenzyl)indolo[6,7:2,'3']-morphinan hydrochloride,
17-allyl-6,7-didehydro-4,5α-epoxy-3-hydroxy-14-(m-chloromethoxyphenyl)1'-(m-chlorobenzyl)indolo[6,7:2,'3']-morphinan hydrochloride,
17-cyclopropylmethyl-6,7-didehydro-4,5α-epoxy-3-hydroxy-14-(o-chloromethoxyphenyl)1'-(o-chlorobenzyl)indolo[6,7:2,'3']-morphinan hydrochloride,
17-cyclopropylmethyl-6,7-didehydro-4,5α-epoxy-3-hydroxy-14-(methoxynaphthalene)1'-(β-methylnaphthalene) indolo-[6,7:2,'3']-morphinan hydrochloride,
17-cyclopropylmethyl-6,7-didehydro-4,5α-epoxy-14-hydroxy-3-benzyloxy-1'-(p-methoxycarbonylmethylphenyl)-indolo[6,7:2,'3']-morphinan hydrochloride.

## Revendications

1. Utilisation de dérivés de morphinane de formule générale I, 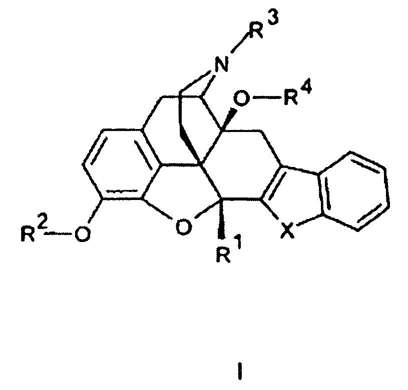 dans laquelle
R¹ représente H, un reste alkyle en C₁ à C₄ ou alcényle en C₂ à C₄,
R² représente H, un reste alkyle en C₁ à C₁₈, avantageusement un reste alkyle en C₁ à C₁₀, un reste alcényle en C₂ à C₁₈, avantageusement un reste alcényle en C₂ à C₁₀, un reste hétérocyclyle ou un reste aryle,
R³ représente un reste alkyle en C₁ à C₁₈, avantageusement un reste alkyle en C₁ à C₁₀, un reste alcényle en C₂ à C₁₈, avantageusement un reste alcényle en C₂ à C₁₀, un reste cycloalkyle en C₃ à C₇, aryle ou hétérocyclyle lié par l'intermédiaire d'un groupe alkylène en C₁ à C₄ ou un reste cycloalkyle en C₃ à C₇, aryle ou hétérocyclyle lié par l'intermédiaire d'un groupe alcénylène en C₂ à C₄,
R⁴ représente H, un reste alkyle en C₁ à C₁₈, avantageusement un reste alkyle en C₁ à C₁₀, un reste alcényle en C₂ à C₁₈, avantageusement un reste alcényle en C₂ à C₁₀, un reste cycloalkyle en C₃ à C₇ ou aryle lié par l'intermédiaire d'un groupe alkylène en C₁ à C₄ ou un reste cycloalkyle en C₃ à C₇ ou aryle lié par l'intermédiaire d'un groupe alcénylène en C₂ à C₄,
et
X représente O ou un groupe NR⁵ dans lequel
R⁵ représente H, un reste alkyle en C₁ à C₁₈, avantageusement un reste alkyle en C₁ à C₁₀, un reste alcényle en C₂ à C₁₈, avantageusement un reste alcényle en C₂ à C₁₀, un reste cycloalkyle en C₃ à C₇, aryle ou hétérocyclyle lié par l'intermédiaire d'un groupe alkylène en C₁ à C₄ ou un reste cycloalkyle en C₃ à C₇, aryle ou hétérocyclyle lié par l'intermédiaire d'un groupe alcénylène en C₂ à C₄,
et/ou leurs énantiomères, leurs diastéréoisomères, des bases ou des sels d'acides physiologiquement acceptables, pour la préparation d'un médicament destiné au traitement :
de douleurs neuropathiques et/ou de l'anxiolyse et/ou de la dépression et/ou de l'incontinence urinaire et/ou des acouphènes et/ou des difficultés auditives et/où de l'épilepsie et/ou de l'obésité et/ou de la cachexie.

2. Utilisation suivant la revendication 1, **caractérisée en ce qu'**on utilise comme dérivés de morphinane
le chlorhydrate de 17-allyl-6,7-didéhydro-4,5α-époxy-3,14-dihydroxy-1'-(o-chlorobenzyl)indolo-[6,7:2,'3']-morphinane,
le chlorhydrate de 17-allyl-6,7-didéhydro-4,5α-époxy-3-hydroxy-14-(m-chlorométhoxyphényl)1'(m-chlorobenzyl)indolo-[6,7:2,'3']-morphinane,
le chlorhydrate de 17-cyclopropyl-6,7-didéhydro-4,5α-époxy-3-hydroxy-14-(o-chlorométhoxyphényl)1'(o-chlorobenzyl)-indolo-[6,7:2,'3']-morphinane,
le chlorhydrate de 17-cyclopropyl-6,7-didéhydro-4,5α-époxy-3-hydroxy-14-(méthoxynaphtalène)1'(β-méthyl-naphtalène)-indolo-[6,7:2,'3']-morphinane,
et/ou
le chlorhydrate de 17-cyclopropylméthyl-6,7-didéhydro-4,5α-époxy-14-hydroxy-3-benzyloxy-1'-(p-méthoxycarbonyl-méthylphényl)-indolo-[6,7:2,'3']-morphinane.
